# EUROPEAN PATENT APPLICATION

(11) **EP 4 729 112 A1**
(43) Date of publication of application: **22.04.2026**
(21) Application number: 25150184.7
(22) Date of filing: 03.01.2025
(51) Int. Cl.: A61N 1/04, A61N 1/36, A61N 1/372, A61N 1/02, A61N 1/375

(54) **NEUROMODULATION DEVICE FOR FUNCTIONAL REHABILITATION AND PAIN THERAPY AND STIMULATION AND CONTROL SYSTEM FOR FUNCTIONAL REHABILITATION AND PAIN THERAPY**

(30) Priority: 21.10.2024 BR 102024021809
(71) Applicant: Franklin da Costa de Paula, Maria Eduarda, 59133-332 Pajuçara - Natal (BR)
(72) Inventor: Franklin da Costa de Paula, Maria Eduarda, 59133-332 Pajuçara - Natal (BR)
(74) Representative: Winter, Brandl - Partnerschaft mbB

(57) **Abstract**

A neuromodulation device for functional rehabilitation and pain therapy (1, 100) is described, comprising a housing body (8, 108) and a housing cover (6, 106). within the housing body (8, 108), at least one electronic plate (7) being positioned, whereby the neuromodulation device for functional rehabilitation and pain therapy (1, 100), the electronic plate (7) comprising at least one control plate (71) equipped with at least one microcontroller for modulation function management, at least one actuator plate (72) equipped with at least one converter and an H-bridge, and at least one Wi-Fi module for connection with at least one sensor (14, 140) in order to forward data to an Artificial Intelligence module (201) and to at least one server (202).

A stimulation and control system for functional rehabilitation therapy and pain therapy is also described, which is capable of collecting biomechanical data from the patient. This data is used for processing and calculations that assist a control subsystem in direct communication with the neuromodulation device for functional rehabilitation and pain therapy, enabling patient monitoring and adjustments of the neuromodulation device during therapy.

## Description

The present invention is related to a neuromodulation device, particularly used for functional rehabilitation therapy and pain therapy in patients by means of the application of electrical stimuli in a non-invasive or extracorporeal manner and to a stimulation and control system for functional rehabilitation therapy and pain therapy, whose system uses the neuromodulation device in conjunction with a control subsystem to apply electrical stimuli in a non-invasive manner allowing patients to regain movements and modulate pain.

### Description of the state of the art

Neuromodulation is a set of medical techniques that, when applied together with neuroscience techniques, is capable of generating physical reactions in people by means of the application of physical properties provided by an equipment or a device.

Usually, neuromodulation is used to treat patients with neuropathic visceral pain who have dysfunctions or damages to the nerve cells of the peripheral or central nervous system, by means of invasive or non-invasive methods.

In this context, at least one neural stimulation device or a neuromodulation device is used, comprising electronic components and electronic circuits capable of operating together to modulate electrical current, voltage, and specific frequencies in such a way that it can influence the nervous activity of the user's neurons by means of electrical or chemical stimuli.

The electric current can be modulated and applied to the neurons and specific nerves of the patients, so that they can generate nerve impulses in the patient's body in a non-voluntary manner. Nerve impulses can be generated for convenient or inconvenient reactions for the patient. In this manner, neuromodulation works together with the brain to decode stimuli into nervous reactions, in such a way that it can transmit the desirable electrical current in the application of neuromodulation, helping to restore neural balance and improve the function of the patient's nervous system.

This application of the modulated current can occur directly at the origin of the unwanted nerve impulses, such as, for example, regions of chronic pain sensation, or can be applied in substantially spatial regions of the patient's body from the point of sensation of local nerve activity. In this way, the modulated electrical current provides a regulation of neural activity with the aim of treating neurological, psychiatric, and other pathological medical conditions such as Parkinson's, chronic pain, depression, epilepsy, among others.

To originate the regime of electrical currents by the neuromodulation device, the presence of a periodic transmission frequency is necessary, which can be either low or high. The range of operation of the working frequency directly influences nerve and neural stimulation; accordingly, the result thereof is one of the variables of the system as a whole that must be managed and administered in a functional therapy.

The neuromodulation device can transmit, as an output signal, a current to the patient's body that must be below the current capacity that the human body can accept as safe. The preference for the type of electrical output current is a design choice aimed at achieving the best configuration for desirable operation during the application of the functional therapy session.

In this regard, there are basically two types of neuromodulation devices: the invasive ones that are implanted in the patient's body, at the location determined by the doctor for neurostimulation, and the non-invasive or extracorporeal ones that are placed on the region of the patient's body to be treated, in external contact with the skin.

Neuromodulation devices designed for applications of invasive technique have a significant disadvantage when compared to neuromodulation devices for applications through non-invasive technique. This is because invasive techniques, being inside the patient's body, present various health risks for the patient, since the installation of the device has a high degree of complexity requiring a medical team for same, and the application thereof can have permanent side effects that, depending on the degree of injury, can become completely irreversible, such as the risk of organ failure, bleeding, infections, allergic rejections, damage to surrounding tissues, loss of motor function of limbs, mood alteration, cognitive dysfunction, and risk of death.

Furthermore, once a neuromodulation device is installed by invasive technique, the patient can hardly remove it in the future because the complications of removal after invading organs, which are more sensitive regions of the human being, involve the same risks presented during the installation thereof, resulting in a high risk of death.

Even though they present many risks, several developments for neuromodulation with invasive technique are known in the state of the art. Document BR112020020867-1, for example, describes a method of therapeutic doses with a neuromodulation device that can apply and adjust the voltage and frequency during the therapeutic session in a patient. In this case, for the operation of this device, it is necessary to use invasive techniques to implant it in the patient's body.

Document US20230284982 also describes invasive techniques of neuromodulation that use electrodes implanted in the patient.

The application of the non-invasive technique is performed extracorporeally to the patient's skin, in such a way that for the application thereof it is only necessary to make superficial contact of the neuromodulation device with the skin. Therefore, the non-invasive neuromodulation device can be easily placed on and removed from contact with the site of the patient's body to be treated.

However, the non-invasive neuromodulation device must transmit electrical power from this device to the patient's body via this contact. This electric current can be direct current, intermittent, pulsed among others. Thus, by means of one or more electrodes connected to the neuromodulation device and arranged on the patient's skin in the region to be treated, the transmission of the electrical current occurs, stimulating neurons that will make involuntary synapses.

The techniques of non-invasive neuromodulation with the use of extracorporeal devices are also known in the state of the art. In this regard, document BR202015033163-6 refers to a handheld neuromodulator that uses transcutaneous electrical stimulation on the patient's skin. This is exclusively a device capable of assisting with overactive bladder dysfunction in way to improve urinary and defecatory symptoms. However, nothing is mentioned about the application of this solution for motor treatment by contracting and relaxing muscles or in the treatment of chronic pain in patients, or even in conjunction with a stimulation system.

Document PI0304175-1 describes a neuro-muscular stimulator consisting of an electronic circuit featuring two or more adjustable amplitude current pulse sources connected to an output transformer, with each source corresponding to a range of variation in the amplitude of the stimulation current pulse. The stimulator to which this document from the state of the art refers is extracorporeal and quite simple in operation, not being managed by systems equipped with any control over the application of the stimulus and, especially, the patient's body response to the applied stimulus. In addition, the application of this stimulation to modulate chronic pain is not described.

Document US10610688 refers to a system and method for managing pain in patients. The system includes sensors configured to detect physiological or functional signals, and a pain analyzer to generate signal metrics from the physiological or functional signals. The pain analyzer also generates weight factors corresponding to the signal metrics. Weight factors can indicate the reliability of signal metrics in representing pain intensity. The pain analyzer generates a score of pain using a plurality of signal metrics and a plurality of weighting factors. The score of pain is sent to a user or a method. The system may further include an electrostimulator to generate and administer closed-loop pain therapy according to the pain score.

Furthermore, although the stimulation systems that control and manage neuromodulators are known, these commercially available stimulation systems known in the literature (Venugopalan, 2020; Shideler, 2020) are limited in terms of data communication, processing, and storage, mainly due to the overall complexity associated with sensor fusion, calculation of joint angles, identification of dynamic models, design and tuning of controllers.

Therefore, given the state of the art, there is a need for a neuromodulation device for functional rehabilitation therapy and pain therapy that is non-invasive, easy and practical to use, and that can be associated with a stimulation and control system for functional rehabilitation therapy and pain therapy, providing a non-invasive therapeutic approach. Furthermore, the system must be capable of offering a motor rehabilitation therapy that allows patients to regain movements and modulate chronic pain, enabling precise control of the standardized electrical stimulus provided from data collection, calculation processing, and generation of modulated pulses that can meet personalized scientific and medical criteria for each patient.

### Objectives of the invention

Thus, the present invention has the objective of providing a neuromodulation device for functional rehabilitation and pain therapy capable of generating electrical pulses and brain wave modulation for extracorporeal action on the patient and, furthermore, enables direct communication with a stimulation and control system for functional rehabilitation therapy and pain therapy by means of a computational interface.

It is also an objective of this invention to provide a stimulation and control system for functional rehabilitation therapy and pain therapy capable of collecting biomechanical and physiological data from the patient that is used for processing and calculations that assist a control subsystem that is in direct communication with the neuromodulation device for functional rehabilitation and pain therapy for patient monitoring and adjustments of the neuromodulation device during therapy.

### Brief description of the invention

In this way, the present invention has as its object a neuromodulation device for functional rehabilitation and pain therapy, comprising a housing body and a housing cover, whereby within the housing body there is arranged at least one electronic plate, the neuromodulation device for functional rehabilitation and pain therapy, the electronic plate comprising at least one control plate equipped with at least one microcontroller for modulation function management, at least one actuator plate equipped with at least one converter and an H-bridge, and at least one Wi-Fi module for connection with at least one sensor in order to forward data to an Artificial Intelligence module and to at least one server.

Another object of this invention consists of a stimulation and control system for functional and pain therapy, comprising:
at least one neuromodulation device for functional rehabilitation and pain therapy, connected to at least one electrode and at least one sensor; and
a control subsystem associated with a management subsystem and in continuous communication with the neuromodulation device for functional rehabilitation and pain therapy and with at least one sensor;
the control subsystem processes input parameters received from the management subsystem and/or at least one sensor to regulate the neuromodulation device for functional rehabilitation and pain therapy for electrostimulation application in at least one receiving user.

### Brief description of the drawings

Figure 1 - illustrates a front perspective view of a first configuration of the neuromodulation device for functional rehabilitation and pain therapy, object of the present invention;
Figure 2 - illustrates a rear perspective view of the first configuration of the neuromodulation device for functional rehabilitation and pain therapy, object of this invention;
Figure 3 - illustrates a side view of the first configuration of the neuromodulation device for functional rehabilitation and pain therapy;
Figure 4 - illustrates a top view of the first configuration of the neuromodulation device for functional rehabilitation and pain therapy, object of the present invention;
Figure 5 - is an exploded view of the first configuration of the neuromodulation device for functional rehabilitation and pain therapy, object of this invention;
Figure 6 - illustrates the electronic plate of the first configuration of the neuromodulation device for functional rehabilitation and pain therapy, object of this invention;
Figure 7 - illustrates a perspective view of a stimulus capture sensor used with the first configuration of the neuromodulation device for functional rehabilitation and pain therapy, object of this invention;
Figure 8 - illustrates a pickup sensor shown in Figure 7;
Figure 9 - illustrates a top view of the stimulus pickup sensor illustrated in Figure 7;
Figure 10 - is an exploded view of the stimulus sensing sensor illustrated in Figure 7;
Figure 11 - illustrates an electrical diagram of the assembly of the electronic plate used with the first configuration of the neuromodulation device for functional rehabilitation and pain therapy, object of this invention;
Figure 12 - illustrates a schematic perspective view of the connection assembly between sensors, neuromodulation device stimulators, and power input network;
Figure 13 - illustrates a power output option of the first configuration of the neuromodulation device object of this invention, which includes the stimulator and the sensor in a single unit;
Figure 14 - is a perspective view of the second configuration of the neuromodulation device for functional rehabilitation and pain therapy, object of this invention;
Figure 15 - is a schematic view of the connection diagram of the second configuration of the neuromodulation device for functional rehabilitation and pain therapy, object of this invention;
Figure 16 - illustrates the shapes of waves modulated by the neuromodulation device for functional rehabilitation and pain therapy, object of this invention;
Figure 17 - is a perspective view of a sensor used in conjunction with the stimulation and control system for functional rehabilitation therapy and pain therapy;
Figure 18 - is a schematic view of the sensor connection diagram used in conjunction with the stimulation and control system for functional rehabilitation therapy and pain therapy;
Figure 19 - illustrates a flow chart of connection between sensors, neuromodulation device stimulators, and power input network, as illustrated in Figure 12;
Figure 20 - illustrates a flow chart of a system configuration for stimulation and control for functional rehabilitation therapy and pain therapy, object of this invention;
Figure 21 - illustrates a flow chart of an alternative configuration of the stimulation and control system for functional rehabilitation therapy and pain therapy, object of this invention;
Figure 22 - illustrates a flow chart of the control subsystem of the stimulation and control system for functional rehabilitation therapy and pain therapy, object of this invention;
Figure 23 - is a schematic view of a configuration of the connection of components for a therapy session as illustrated in Figure 20;
Figure 24 - is a schematic view of an alternative configuration for connecting the components for a therapy session, as illustrated in Figure 21;
Figure 25 - illustrates a scheme of connections between the neuromodulation device, according to the second configuration thereof, the sensor, and other components of the stimulation and control system for functional rehabilitation therapy and pain therapy;
Figure 26 - illustrates a schematic flow chart of the use of the stimulation and control system for functional rehabilitation therapy and pain therapy, object of this invention; and
Figure 27 - illustrates the varied geometric shapes of the neuromodulation device, the sensor, and the stimulator.

### Detailed description of the invention

The objects of the invention presented here are related to the area of Neuro prosthetics that uses functional electrical stimulation through neuromodulation, conceived by non-invasive or extracorporeal techniques to recover and rehabilitate motor functions of patients who need functional therapy, as well as to treat chronic pain in patients.

A neuro prosthetic is understood as being a physical device capable of interacting with the patient's nervous system, sending or receiving signals to the patient's brain through electrostimulation applied by non-invasive means.

In this regard, the patient, also identified as the receiving user, is the person who receives the electrostimulation in a therapeutic session or functional therapeutic session. The physician or health professional is the person responsible for configuring, controlling, and directing the input and output data of the system during the therapeutic session, as will be described in detail below.

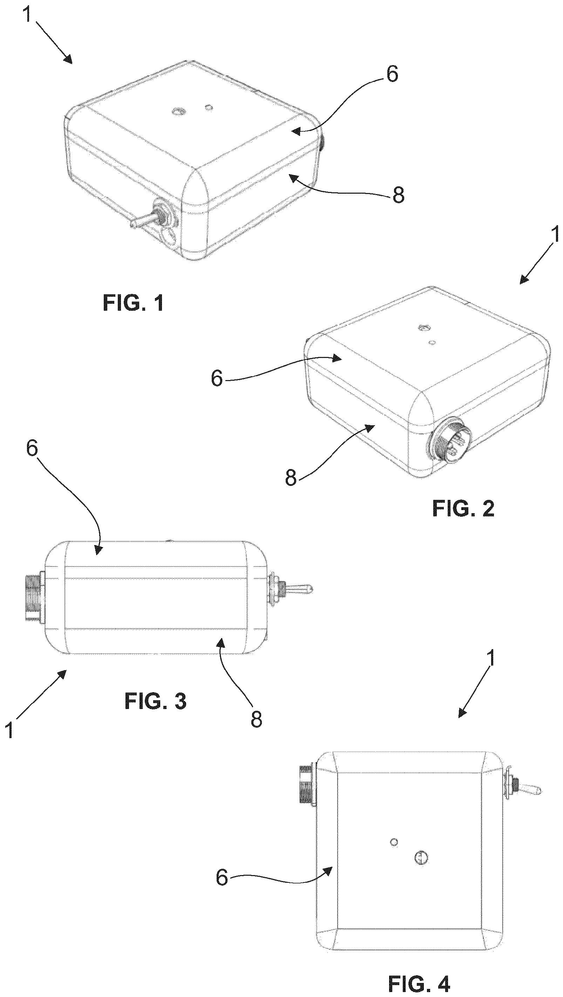

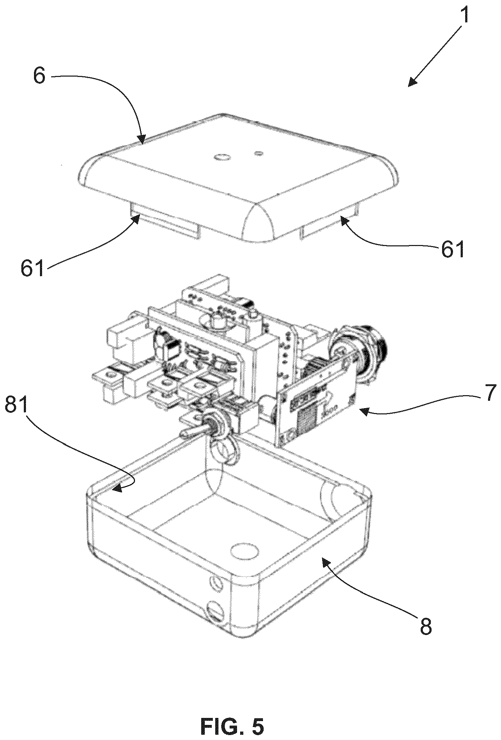

According to a first configuration of the neuromodulation device for functional rehabilitation and pain therapy illustrated in Figures 1 to 10, this neuromodulation device 1 comprises a housing formed by a housing body 8 and a housing cover 6, within which at least one electronic plate 7 is arranged (Figure 5). This housing can be made of polymer, ceramic, wood, metal, fabric, rubber, among other materials or a combination of materials. The junction of housing body 8 with the housing cover 6 is made preferably by the engagement of flaps 61 arranged on housing cover 6 into recesses 81 present in the housing body 8; however, other types of junctions can be used, such as fastening elements (glue, screw, nail, clamps, etc.), mechanical interference, press-fit connections, among other possibilities that allow housing cover 6 to be firmly associated with the housing body 8.

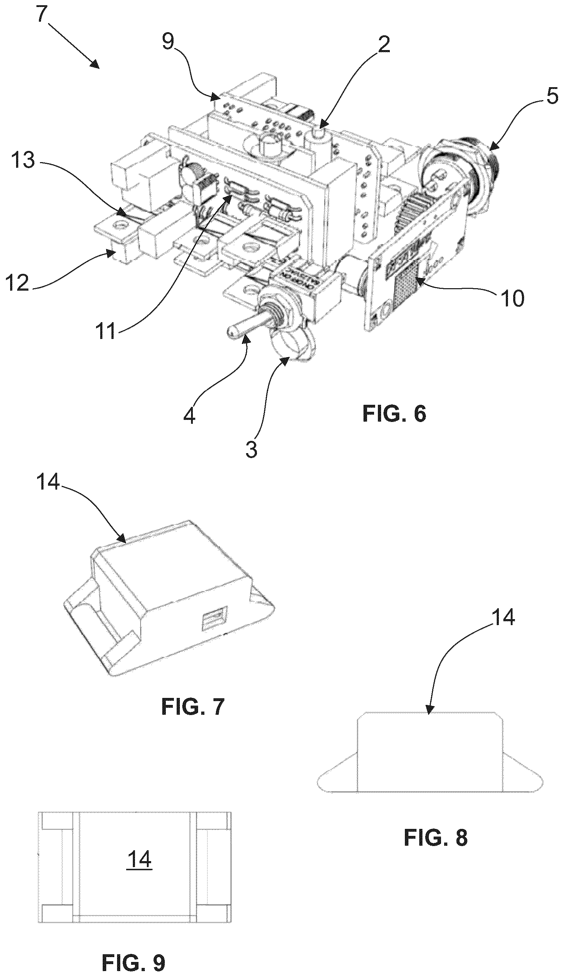

The electronic plate 7 illustrated in Figures 5 and 6 is the electronic system of the neuromodulation device 1. This electronic system is responsible for the formation of waves of electrical stimuli that are applied to the patient by means of neuromodulation device 1. This electronic plate 7 is substantially composed of at least one processor 9, at least one transformer 12, at least one light indicator 2, at least one activation switch 4, at least one power input adapter 3, at least one power output adapter 5, at least one current converter so that either direct current or alternating current can be used, at least one transistor 13, at least one electrical resistor 11, at least one capacitor, at least one voltage booster circuit, at least one voltage regulator circuit, at least one battery charger circuit, and at least one communication connection module 10, responsible for communication between the management system and the control system, as will be further described.

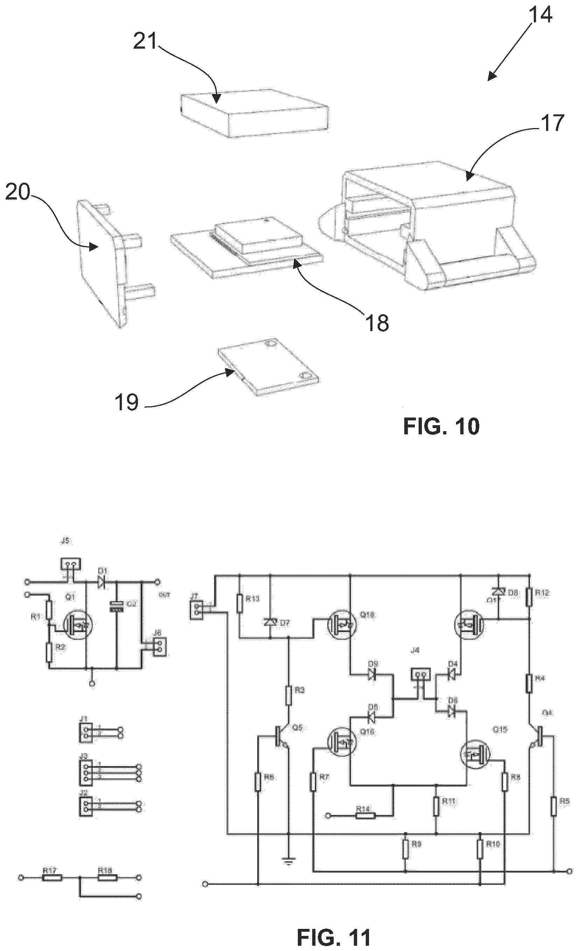

The arrangement of these components of electronic plate 7 follows the configuration illustrated in Figure 11.

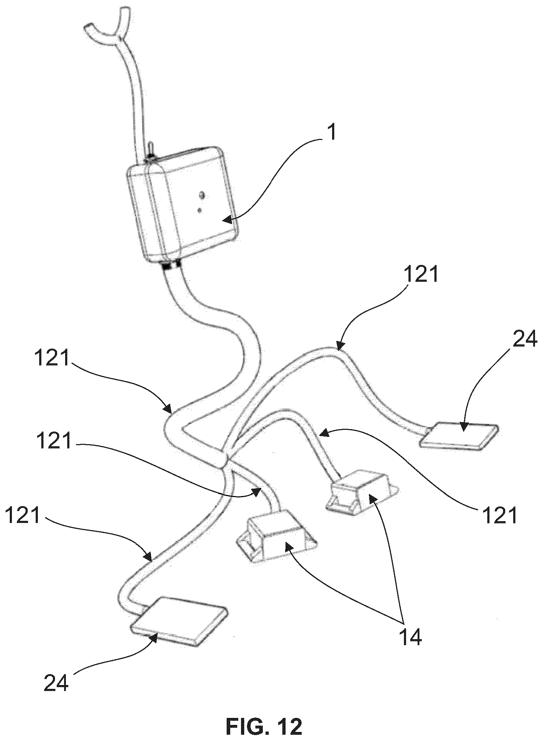

As illustrated in Figure 12, the neuromodulation device 1 applies the electrical stimuli waves to the patient by means of at least one electrostimulator or electrode 24, which is positioned on the patient's skin at the determined location, in an extracorporeal manner, i.e., without being inserted into the patient's body. In this manner, the electrostimulators or electrodes 24 transmit the energy modulated by the neuromodulation device 1 to the patient in a non-invasive manner.

The electrostimulators or electrodes 24, illustrated in Figure 12, are made of a non-insulating porous membrane, and their connection to the neuromodulation device 1 can be made via cables 121 or through wireless communication. These electrostimulators or electrodes 24 are positioned in the region of the patient's body to be treated by a functional therapeutic session or a chronic or not pain therapy. The energy charge in the shape of electrical waves from the neuromodulation device 1 is transmitted to the patient's body region, preferably in a pulsating form, acting on the muscular tissues and the nervous system of that patient.

Along with the electrostimulators or electrodes 24, sensors 14 are also positioned on the patient's body, responsible for capturing the reaction of the patient's body after receiving the modulation of electrical energy provided by the neuromodulation device 1 through the electrostimulators or electrodes 24.

According to Figures 9 and 10, the sensors 14 comprise a hollow body 17 and a portion of closure 20, made of plastic, metal, rubber, wood, among other materials. Inside hollow body 17 there are arranged at least one transmitting module 19, at least one sensory module 18, and at least one battery 21. The hollow body 17 serves as protection for these components, without interfering with their respective functions. The junction of the closing portion 20 with hollow body 17 is made by screws, nails, or mechanical fitting.

The battery 21 supplies power to the sensor 14 in case it is not connected by cable 121 with the neuromodulation device 1. Sensory module 18 has the function of capturing biomechanical and physiological data that these sensors capture from the patient's body reactions, while transmitting module 19 is responsible for facilitating communication between sensor 14, neuromodulation device 1, and the electrostimulation system.

When in use, at least one sensor 14 is positioned on the patient's skin, that is, also in a non-invasive manner, and close to the region undergoing electrostimulation. Sensor 14 captures the biomechanical and physiological data that this type of sensor captures and transforms it into binary data that is sent to a control system 27. The biomechanical and physiological data that sensor 14 captures from the patient can include temperature, blood pressure, movement, angles, contractions, brain signals, humidity, deformation, among other data that the patient can generate with their body.

Sensors 14 can work simultaneously with more than one type of biomechanical and physiological data collection, or they can collect at least one biomechanical and physiological data from the patient. The type of sensor 14 to be used is a choice of the controlling user; however, the operation of this sensor 14 in conjunction with the electrostimulation system that will be detailed below does not change.

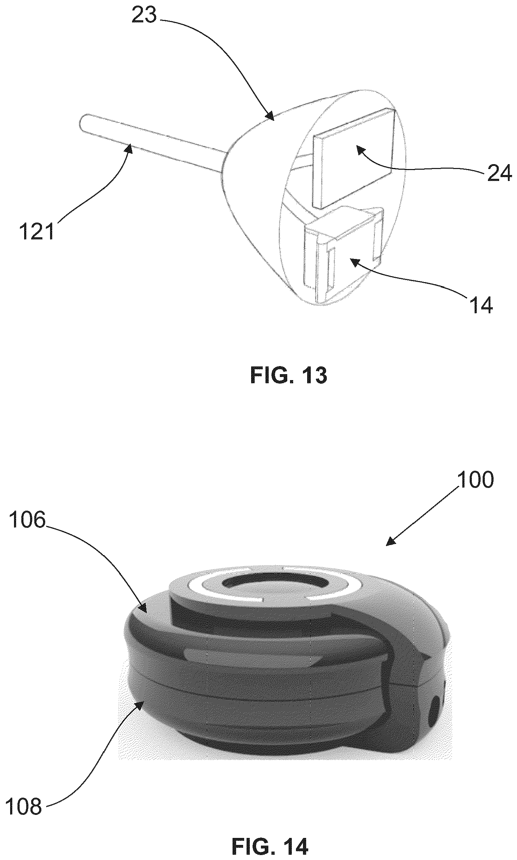

Sensors 14 and electrodes 24 can be separate or, optionally, in a joint configuration housed in the same housing 23, as illustrated in Figure 13. In the joint configuration, each piece of equipment will be responsible for the same function determined in the separate configuration, meaning the configuration does not alter the functionality of each of these pieces of equipment, nor does it alter the outcome of the therapeutic session.

The manner in which sensors 14 communicate with the neuromodulation device 1 and with the electrostimulation system can be by means of data transmission via wire 121 or wirelessly. Wireless technology can be a Wi-Fi connection system, mobile data network, Bluetooth, infrared, among other types of technologies that enable wireless data transmission.

In a second configuration illustrated in Figures 14 to 16, the neuromodulation device for functional rehabilitation and pain therapy or neuromodulation device 100 comprises a casing formed by a housing base 108 and a housing cover 106, said casing internally receiving electronic plate 7.

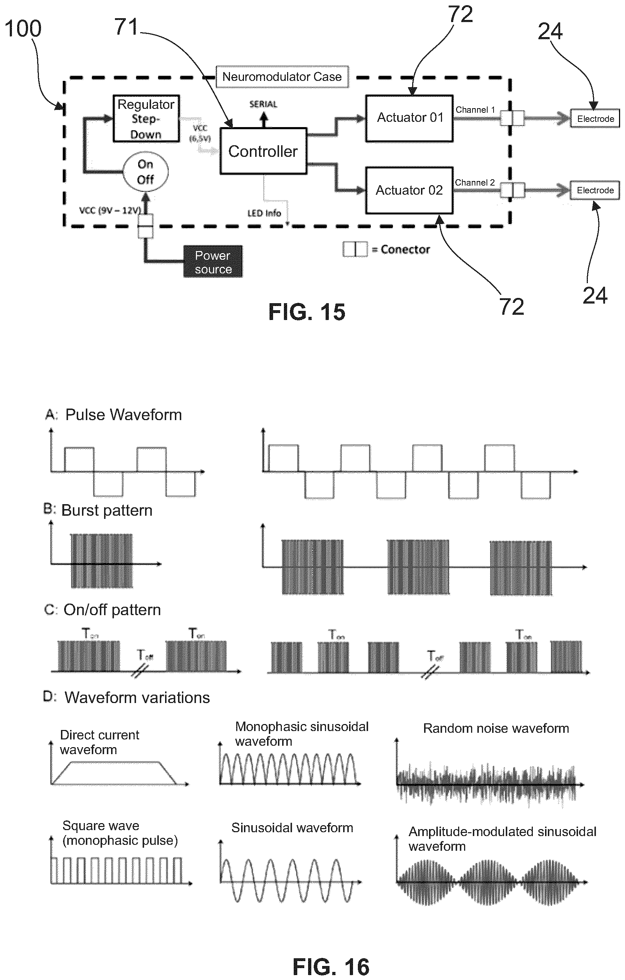

The primary goal of neuromodulation device 100 is to provide a standardized electrical stimulus that meets criteria tailored to the patient. To this end, and as illustrated in Figure 15, electronic plate 7 of the neuromodulation device 100, in addition to comprising the components described for the first configuration of the neuromodulation device 1, further comprises a controlling plate 71 and at least one actuator plate 72. Control plate 71 comprises a microcontroller and auxiliary components, while actuator plate 72 comprises a Boost Converter having the function of raising the DC current voltage from the input of 9-12V to values between 200 - 600V and an H-Bridge whose function is to reverse the direction of the current as needed, i.e., in the case of AC current supply. Preferably, neuromodulation device 100 is formed by a plate controller 71 and by two plate actuators 72 that communicate with electrodes 24 to provide electrical stimuli to the patient.

Further, the neuromodulation device 100 comprises a Wi-Fi module (not shown) embedded in its electronic plate 7. Neuromodulation device 100 connects to an available Wi-Fi network, generally by means of an initial setup process that may involve network selection, password entry, etc. Once configured, neuromodulation device 100 establishes a Wi-Fi connection with a router or access point. The Wi-Fi module allows the creation of various applications for Internet of Things (IoT) designs, remote access, web servers, and dataloggers, enabling the connection of the neuromodulation device 100 to wireless networks and allowing the remote management thereof via the web, as will be described later.

The microcontroller coupled to the plate control unit 71 is responsible for managing all the main functions of the neuromodulation device 100, both the PWM (Pulse Width Modulation) and the frequency of wave generation at the output, as well as communicating with the Wi-Fi module. Thus, in terms of wave generation, neuromodulation device 100 generates square signals with customized frequencies and in an isolated manner, as well as sinusoidal signals using the Boost Converter mentioned above. Figure 16 illustrates the possible types of waves generated by the neuromodulation device 100 due to the electronic arrangement described herein.

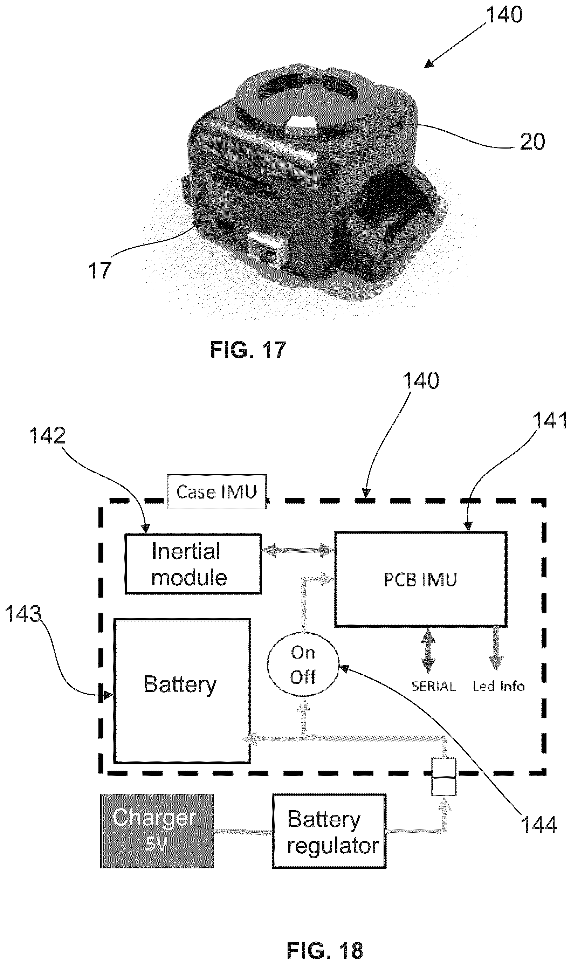

As already mentioned, neuromodulation device 100 is responsible for generating the electrical pulses and the modulation of brain waves. To this end, the neuromodulation device 100 works in conjunction with at least one electrode 24 and at least one sensor 140 (Figure 17). The neuromodulation device 100 is powered by rechargeable batteries, which enables portability and continuous use by the patient, but, optionally, neuromodulation device 100 may comprise a wireless charging system.

Just as in the first configuration, electrodes 24 come into direct contact with the patient's skin and have the function of transmitting the electrical pulses generated by the neuromodulation device 100 to the patient's body, in the position determined by the controlling user or healthcare professional 25, ensuring a non-invasive stimulation that is precise and effective.

The second configuration of sensor 140, similarly to the first configuration of this component, collects biomechanical and physiological data from the patient emitted by the patient's body during the use of neuromodulation device 100. This biomechanical data is used for processing and calculations of joint angles, aiding the decision-making of an Artificial Intelligence (Al) 201 module, which will be explained later.

As illustrated in Figures 17 and 18, in its second configuration, the electronic part of sensor 140 is divided into two main components: a control board 141 composed of a voltage regulator, adjustment resistors, and microcontroller, and an inertial module 142 connected to control board 141. In addition to these components, microcontroller activation resistors and an information LED are also used. The microcontroller is responsible for communicating with the Wi-Fi module to receive the commands and start streaming the data from sensor 140. Inertial module 142 is used to obtain the references from the accelerometer to determine the position of that sensor, while the information LED has the function of indicating whether it is on or off. In this case, the LED can also be an RGB LED so that some functionalities like on, ready for connection, discharging, etc., can be programmed.

The 140 sensor is powered by a 1000mAh battery 143. In addition, a button 144 is used to turn the system on and off, along with a simple LED to indicate the on status. The charging of battery 143 is done via a wired connector, with the possibility of using a micro-USB charger and an external lithium battery charger module.

Neuromodulation device 1, 100 is the main component of the stimulation and control system for functional rehabilitation therapy and pain therapy that is also the object of this invention. By means of neuromodulation device 1, 100, the stimulation and control system is capable of collecting biomechanical and physiological data from the patient and processing it in order to adjust the functions of the neuromodulation device 1,100 for a functional or chronic pain therapeutic session, whether personalized or not, according to the needs and, primarily, according to the biomechanical responses of the patient's body.

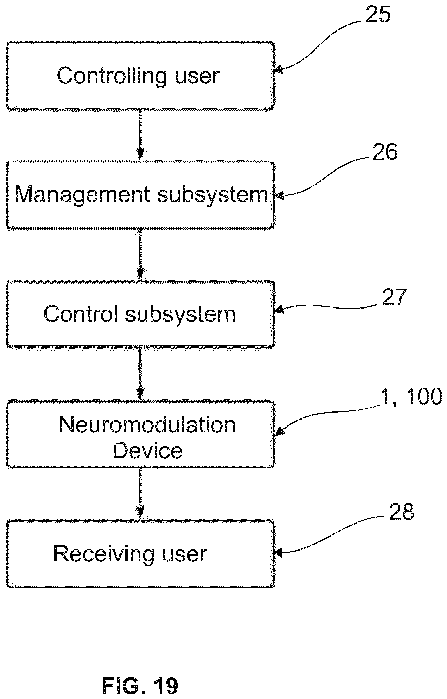

As can be seen in Figure 19, in general terms, the stimulation and control system for functional rehabilitation therapy and pain therapy, object of this invention, comprises a management subsystem 26 that is accessed by at least one controlling user or medical user 25 so that, by means of a control subsystem 27, it acts on at least one neuromodulation device 1, 100 to deliver to at least one patient or receiving user 28 a functional therapeutic session or a pain therapeutic session customized according to the parameters of the patient or receiving user 28.

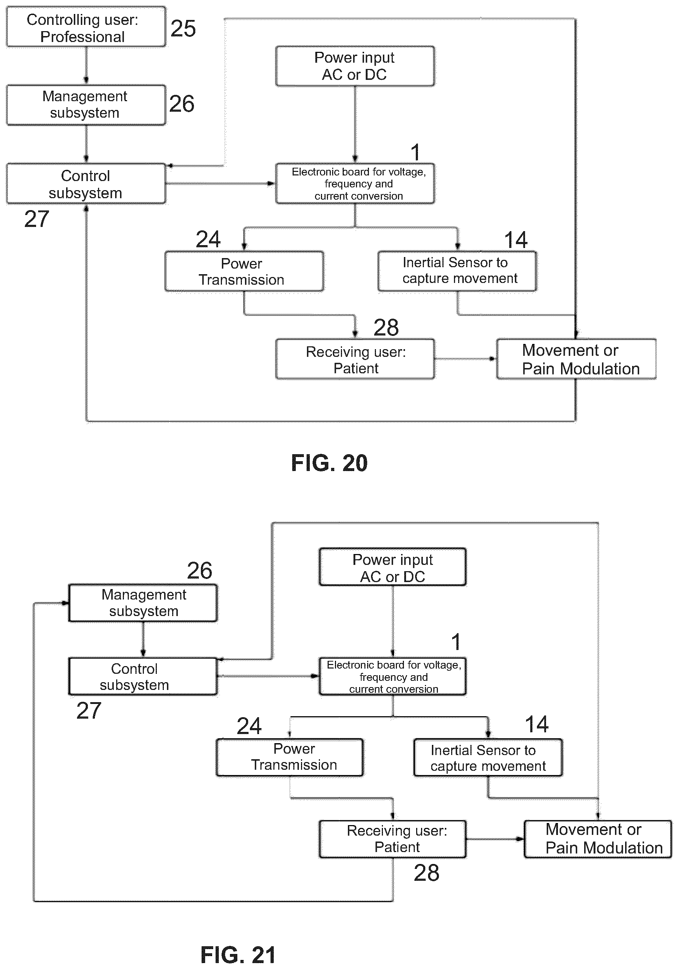

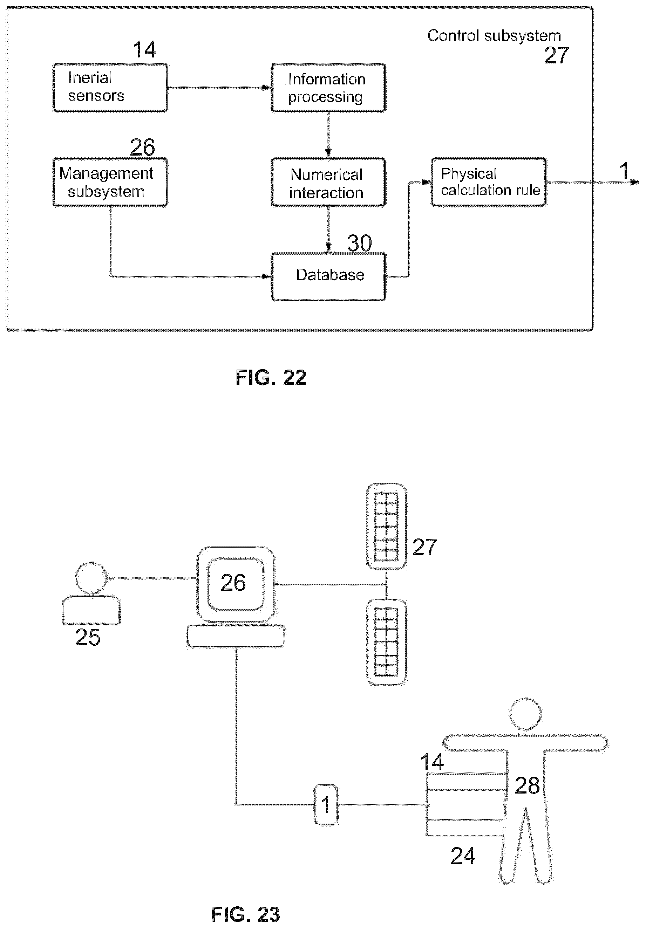

In this regard and as can be seen in Figures 20, 21, and 22, control subsystem 27 uses input parameters that can be provided by the management subsystem 26, which received them from access by a controlling user or healthcare professional 25, through feedback from data originating from at least one sensor 14, or by querying a database 30 (Figure 22). Optionally, as can be seen in Figure 21, the input parameters used by control subsystem 27 originate from management subsystem 26, which obtains them from data feedback from at least one sensor 14 or from information entered into this management subsystem 26 by the receiving user or patient 28 themselves.

These input parameters used by control subsystem 27 consist of recipient or patient 28 data, such as: gender, age, weight, height, medical information regarding mobility and pain, setup data for neuromodulation device 1, information about the quantity and duration of therapeutic sessions, among others.

Control subsystem 27 provides, based on the received and processed parameters, the setup or adjustment of neuromodulation device 1 so that it modulates the signals and applies electrostimulation to patient 28. The communication between control subsystem 27 and neuromodulation device 1 occurs through the communication connection module 10 arranged on electronic plate 7 of neuromodulation device 1. For modulation to occur, it is necessary for neuromodulation device 1 to be connected to at least one power input, which can come from a local electrical grid or by means of batteries of neuromodulation device 1. The electric current provided for neuromodulation device 1 can be continuous current CC or alternate current AC, whereby the current output can be converted to the type that is more convenient to obtain the best result in the therapeutic session.

Once neuromodulation device 1 is activated by control subsystem 27, the transmission of energy from the electrical waves or electrostimulation is delivered to the receiving user or patient 28 through electrodes 24 that are positioned on the skin of patient 28, in an extracorporeal and non-invasive manner, in the region of the patient's body 28 wherein the functional or motor therapeutic session will be conducted, i.e., to perform movement of the patient's body 28 or the therapeutic session for pain modulation, whether chronic or not, of patient 28.

During the application of electrical stimulation on patient 28, sensors 14 capture external biomechanical and physiological data or biomechanical and physiological data generated by the body of patient 28, transform this information into binary data that is sent to the control subsystem 27 as feedback. Control subsystem 27 can store this information in at least one database 30 or transmit it to management subsystem 26 so that it delivers the information to the controlling user or doctor 25 for evaluation. These biomechanical and physiological data that this type of sensor captures can include movement, angles, contractions, brain signals, among other data generated by the body of patient 28 during and/or immediately after the end of the therapeutic session.

In addition, sensors 14 can establish input parameters for control subsystem 27 so that the stimulation and control system can begin operating. Using these parameters is a choice that controlling user 25 can select in management subsystem 26, with the objective of improving the efficiency of the therapeutic session.

Therefore, the input parameters for the setup of neuromodulation device 1 can be configured through manual input by the controlling user or healthcare professional 25, by querying a database 30 connected to the control subsystem 27, or by the feedback generated by the sensors 14.

Any input data entered into the stimulation and control system passes through control subsystem 27. The data provided by control subsystem 27 are sent to neuromodulation device 1 for the regulation of parameters for the transmission of energy or electrostimulation to the receiving user or patient 28. Thus, control subsystem 27 is responsible for correlating, balancing, and parameterizing the electric current, voltage, type of electric current, frequency, and movement angles.

In this regard, the operation of control subsystem 27 is governed by a mathematical and physical equation that weighs the input parameters in such a way that the output of electrical energy by the neuromodulation device 1 is as efficient as possible for the desired therapeutic session. Basically, control subsystem 27 receives at least one physical input parameter and transforms it into an output parameter for neuromodulation device 1, so that this neuromodulation device 1 can read and transmit energy to patient 28.

Thus, as detailed in Figure 22, the manner of operation of control subsystem 27 is formed by two lines of action. In a first line, the information reaches control subsystem 27 through sensors 14. In this case, control subsystem 27 processes the information received that is sent for a numerical interaction. The information, after the numerical interaction, can be included in database 30, and follows the physical calculation rule until it is delivered to neuromodulation device 1.

In the second line of action, the information reaches control subsystem 27 through the management subsystem 26 activated by a controlling user 25. In this second option, management subsystem 26 queries data from database 30 to send the information to control subsystem 27. Once in control subsystem 27, this information goes through the physical calculation rule to be delivered to neuromodulation device 1.

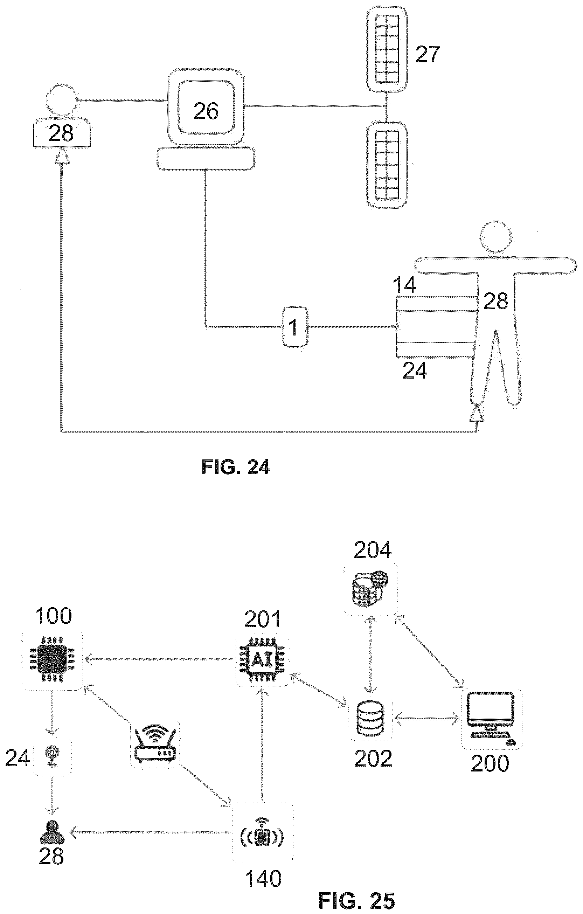

These stimulation and control system options are schematically illustrated in Figures. 23 and 24. As illustrated in Figure 23, the stimulation and control system can be used by a controlling user 25, unlike receiving user 28, who activates the management subsystem 26 that communicates with control subsystem 27 in order to send parameters to neuromodulation device 1 so that it applies electrostimulation to the receiving user 28 through electrodes 24. Simultaneously with the application, sensor 14 captures biomechanical data from recipient user 28 and sends it to control subsystem 27 via the neuromodulation device 1 or directly to control subsystem 27, as a data feedback.

Figure 24 illustrates the same operation described above; however, controlling user 28 is the same receiving user 28 who receives the benefits generated during the therapeutic session.

In an alternative configuration that uses the second configuration of neuromodulation device 100, as illustrated in Figure 25, the stimulation and control system comprises at least one electrode 24 and at least one sensor 140 positioned on the patient's body, in a non-invasive manner, ensuring accuracy in delivering electrical stimuli and capturing biomechanical data of patient 28, respectively.

Neuromodulation device 100 is connected to electrodes 24 by wires and, through the electrodes 24, neuromodulation device 100 applies the stimuli to patient 28 according to a customized schedule. Further, neuromodulation device 100 is also connected to sensor 140 via a Wi-Fi network. Neuromodulation device 100 and sensor 140 communicate with an access computer 200 via the Wi-Fi network. On access computer 200 are management subsystem 26 and control subsystem 27.

Sensor 140 captures biomechanical data from patient 28 during the therapy session, these biomechanical data are then processed by an Artificial Intelligence (Al) module 201 located on a server 202. Server 202 is responsible for ensuring accuracy in calculations related to stimulation. This allows the generation of predictive control actions, anticipating the behavior of the system based on the data received in real time. In addition, the server also provides an efficient loT connection, optimizing the exchange of data between devices.

In access computer 200 are the management subsystem 26 and the control subsystem 27, thus, access computer 200 communicates with server 202 and with cloud server 204, allowing the management or administration of patients, the connection with neuromodulation devices 100, and the adjustment of stimulation parameters via a computational interface.

The computational interface or user interface is a web page, a mobile or desktop application, with a set of buttons that allow the user to configure and control neuromodulation device 100 remotely, manage one or more patients, personal data, clinical data, and respective protocols. The user interface also provides biometric information of the patients to the healthcare professionals.

The functional motor therapeutic session for patients 28 with the use of the stimulation and control system for functional and pain therapy and neuromodulation device 1, 100 occurs through the neuromodulation of electrical current at a specific frequency that directly targets the patient's nervous system, causing the patient's muscle tissues to respond with relaxation and contraction.

In order for the patient to be able to move their limbs, neuromodulation device 1,100 promotes the contraction and relaxation of the patient's 28 muscles through logical sequences and electrostimulation, which are responsible for the ideal moment capable of generating movement of limb angles. This logical sequence is cyclically repeated, in a constant or variable manner, depending on the information of angle, stream, voltage, frequencies, and results captured and presented by sensors 14, 140.

To perform the control of the movement of patient's 28 limbs involuntarily to patient's 28 body, the stimulation and control system for functional and pain therapy uses an input data in its control subsystem 27 that establishes the opening angle and stopping of the movement. This input data can be entered into control subsystem 27 directly by management system 26 or by feedback from the biomechanical and physiological data collected by sensors 14, 140. In this way, management system 26 is able to present to control user 25 an arrangement of information such as input and output parameters, logical sequences performed and future, two-dimensional or three-dimensional visualization of the patient's 28 members, and biomechanical information captured by sensors 14, 140.

The stimulation and control system for functional therapy and pain is also used for therapeutic sessions to relieve the sensation of visceral pain or chronic pain in patients 28, and this function may be relatively a consequence of the contraction and relaxation of muscle tissues. It is emphasized that electrostimulation is a therapeutic technique widely used in pain treatment, utilizing controlled electrical impulses to stimulate muscle or nerve tissues. Its main objective is to modulate neural activity, promoting pain relief.

In the case of therapeutic sessions for relief of visceral pain sensation or chronic pain, the stimulation and control system for functional therapy and pain manages the pain that patient 28 has in their body, positioning electrodes 24 and sensors 14, 140 at any points on the skin of patient 28 and transmitting low-intensity electrical impulses to stimulate the peripheral nerve fibers. The frequency and intensity of the stimuli are adjusted according to the type and severity of the pain, always under the guidance of a trained health professional.

This action mechanism of electrostimulation is mainly based on the Gate Control Theory. This theory proposes that electrical impulses block the transmission of pain signals to the brain by activating larger diameter nerve fibers, which effectively "closes" the pain gate in nerve pathways.

Thus, the control of visceral pain sensation technically occurs through neuromodulation device 1, 100, which provides current and frequency neuromodulation within a specific working range that prevents the patient's nervous system 28 from transmitting to the brain the local disturbances generated in pain sensation, in such a way that the communication of the nervous system with the patient's 28 brain is inhibited.

For the stimulation and control system for functional and pain therapy to systematically act on various types of human bodies of different patients, the neuromodulation device 1, 100 works preferably in a frequency range, current, voltage, and angle. The operating range of the device corresponds to the different electrical stimulations. However, even if the electrical stimulations change, the physical properties remain, in a manner that the control subsystem 27 remains unchanged.

In this sense, the energy output of the neuromodulation device 1, 100 can vary within the range where the voltage is preferably between 1 and 500 V, where the electric current is between 0 and 1.0 A, and where the frequency is between 0 and 5000 Hz. In this context, the movement angle can be from 0° to 360° in any axis where the patient 28 can perform movements.

These output parameters can be pre-established or post-established in these presented ranges, and the combination between the iterations performed among the parameters can substantially operate in probabilistic settings. The settings present the composition in which one parameter is fixed and the others vary, or in which two parameters are fixed and the other two vary, or in which three parameters are fixed and the other varies, or even in which all four can be varying at the same time.

The control of the variation of these parameters can be done automatically by the control subsystem 27 or manually by the intervention of the controlling user 25 during the therapeutic session period.

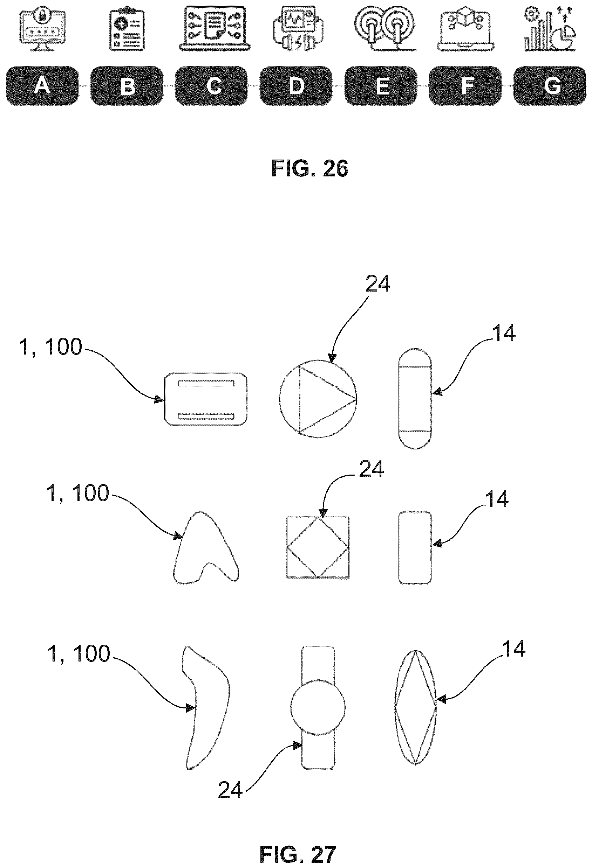

The use of the stimulation and control system for functional and pain therapy by the user is done in a simple manner, as illustrated in Figure 26. In step A, the user, who can be the controlling user 25 or the receiving user 28, logs into the system using their provided credentials. Next, in step B, the user follows the protocols to set up the clinical data of the recipient user or patient 28, including filling out medical forms and recording other relevant information. In step C, the user accesses the neuromodulation device section 1, 100, wherein they adjust the specific parameters of the therapeutic session that patient 28 will receive (functional therapeutic session for mobility or therapeutic session for pain), ensuring that all settings are appropriate for the therapeutic needs of patient 28. Step D involves setting up neuromodulation device 1, 100, connecting it to the internet, and adjusting the technical parameters according to the treatment specification. In step E, the user then positions electrodes 24 and sensors 14, 140 on the patient's body 28 following the guidelines detailed in the manual to ensure the accuracy and effectiveness of the treatment. With everything prepared, in step F the user activates the stimulation and control system for functional and pain therapy and monitors the performance of the neuromodulation device 1, 100 during the therapeutic session, tracking in real-time the patient's progress and reactions. After the completion of the therapeutic session, the stimulation and control system for functional and pain therapy generates a bio-response report in step G, which provides a detailed analysis of the collected data, allowing the user to assess the effectiveness of the treatment and adjust future procedures as necessary.

Both the stimulation and control system for functional and pain therapy, as well as neuromodulation device 1, 100 for functional and pain therapy, use non-invasive techniques, requiring only the contact of electrodes 24 and inertial sensors 14, 140 with the patient's skin to enable the modulation of electrical current, frequency, voltage, and angle, and to capture the biomechanical and physiological information of the patient that this type of sensor captures.

Non-invasive techniques promote various benefits for the patient's health, such as the reduction or even absence of damage to nerve cells, bleeding, infections, allergic rejections, damage to surrounding tissues, loss of motor function in limbs, mood changes, cognitive dysfunctions, and risk of death. The use of the present invention maintains the integrity of the patient's body. This is because the neuromodulation device 1, 100 operates through superficial contact with the patient's skin made by electrodes 14 and sensors 14, 140, which, as already described, are positioned in any superficial regions of the patient's body.

Another advantage of the stimulation and control system for functional and pain therapy, like the neuromodulation device 1,100 for functional and pain therapy, is the portability of the neuromodulation device 1,100 and the possibility of remote communication of this neuromodulation device 1,100 with the stimulation and control system for functional and pain therapy for the necessary setup adjustments and obtaining return information.

In this way, the use of the stimulation and control system for functional and pain therapy, such as neuromodulation device 1, 100, can result in the possible recovery of nerves, muscle tissues, and reconditioning of the nervous system, in addition to prolonged relief for the patient's 28 visceral or chronic pain.

Specifically in relation to neuromodulation device 1, 100, the electrodes 24 and sensors 14, 140, these can come in various geometric shapes, providing greater freedom for use in the most convenient format for the profile of the receiving user or patient 28 (adult, elderly, children) and for the need of the area of the patient's body 28 to receive the electrodes 24 and sensors 14, 140, whether large or small. In this regard, Figure 27 illustrates examples of formats for neuromodulation devices 1, 100, electrodes 24, and sensors 14, 140; however, many other formats can be used as long as they are functional.

Still in relation to neuromodulation device 1, 100, this can present one or more information inputs, one or more information outputs, one or more light indicators, one or more graphical displays of both instantaneous information and performed parameters.

Having described a preferred embodiment example, it should be understood that the scope of the present object encompasses other possible variations, being limited only by the content of the appended claims, including possible equivalents.

## Claims

1. Neuromodulation device for functional rehabilitation and pain therapy (1, 100), comprising a housing body (8, 108) and a housing cover (6, 106), within the housing body (8, 108) there is arranged at least one electronic plate (7), the neuromodulation device for functional rehabilitation and pain therapy (1, 100) being **characterized by** the fact that the electronic plate (7) comprises at least one control plate (71) equipped with at least one microcontroller for managing modulation functions, at least one actuator plate (72) equipped with at least one converter and an H-bridge, and at least one Wi-Fi module for connection with at least one sensor (14, 140) in order to forward data to an Artificial Intelligence module (201) and to at least one server (202).

2. Neurostimulation device, according to claim 1, **characterized by** the fact that it is connected to at least one electrode (24) to provide customized electrical stimulation to at least one recipient user (28), with at least one electrode (24) being positioned extracorporeally on the recipient user (28).

3. Neuromodulation device, according to claim 1, **characterized by** the fact that the microcontroller is coupled to the plate controller (71) to manage the modulation of pulses, frequency, and output wave generation.

4. Neuromodulation device, according to claim 1, **characterized by** the fact that it is handheld.

5. Stimulation and control system for functional and pain therapy, **characterized by** comprising:
at least one neuromodulation device for functional rehabilitation and pain therapy (1, 100) as defined in claims 1 to 4, connected to at least one electrode (24) and to at least one sensor (14, 140); and
a control subsystem (27) associated with a management subsystem (26) and in continuous communication with the neuromodulation device for functional rehabilitation and pain therapy (1, 100) and with at least one sensor (14, 140);
the control subsystem (27) processes input parameters received from the management subsystem (26) and/or at least one sensor (14, 140) to regulate the neuromodulation device for functional rehabilitation and pain therapy (1, 100) for the application of electrostimulation in at least one receiving user (28).

6. System, according to claim 5, **characterized by** the fact that the input parameters of the control subsystem (27) can come from a database (30).

7. System, according to claim 5, **characterized by** the fact that the input parameters provided by the management subsystem (26) originate from feedback from data by at least one sensor (14, 140) or information entered into the management subsystem (26) by the receiving user (28) or by a controlling user (25).

8. System, according to claim 5, **characterized by** the fact that communication between the control subsystem (27) and the neuromodulation device for functional rehabilitation and pain therapy (1, 100) is carried out by means of a communication connection module (10).

9. System, according to claim 5, **characterized by** the fact that the neuromodulation device for functional rehabilitation and pain therapy (1, 100) is connected to at least one sensor (14, 140) via a Wi-Fi network.

10. System, according to any of the preceding claims, **characterized by** the fact that the input parameters are entered into the management subsystem (26) by the controlling user (25) or receiving user (28) by means of an interface.

11. System, according to claim 10, **characterized by** the fact that at least one sensor (14, 140) collects biomechanical parameters from the receiving user (28) for processing in an Artificial Intelligence module (201).
